# FASCICULE DE BREVET EUROPEEN

(11) **EP 0 720 839 B1**
(45) Date de publication et mention de la délivrance du brevet: **08.03.2000**
(21) Numéro de dépôt: 95420367.5
(22) Date de dépôt: 26.12.1995
(51) Int. Cl.: A61F 2/36, A61F 2/40

(54) **Tige fémorale à profil vrille pour prothèse de hanche**
Femurschaft mit verwringtem Profil für Hüftprothese
Femoral shaft with twisted profile for hip prosthesis

(30) Priorité: 03.01.1995 FR 9500141
(43) Date de publication de la demande: 10.07.1996
(73) Titulaire: TORNIER SA, 38330 Saint-Ismier (FR)
(72) Inventeur: Van de Velde, Denis, F-59300 Valenciennes (FR); Roumazeille, Bertrand, F-59320 Escobecques (FR); Chatelan, Jean-Louis, F-33420 Guillac (FR); Tabutin, Jacques, F-06110 Le Cannet (FR); Bejui, Jacques, F-69006 Lyon (FR); Fessy, Michel-Henri, F-69006 Lyon (FR); Gleyze, Pascal, F-68000 Colmar (FR); Bar, Pierre, F-59800 Lille (FR)
(74) Mandataire: Myon, Gérard Jean-Pierre

(56) Documents cités:
- EP-A- 0 128 036
- EP-A- 0 373 011
- EP-A- 0 629 386
- WO-A-91/18560
- WO-A-94/08534
- FR-A- 2 639 821
- FR-A- 2 676 914
- US-A- 4 030 143

## Description

La présente invention a trait à une tige pour prothèse, selon le préambule de la revendication 1, et notamment à une tige fémorale de prothèse totale de hanche présentant une géométrie particulière pour optimiser les zones d'appui dans le canal médullaire, tout en respectant la structure de l'os.

Une tige selon le préambule de la revendication 1 est connue dans le document FR-A-2 676 914.

La tige suivant l'invention est destinée à être introduite sans ciment dans le canal médullaire d'un os long et notamment d'un fémur.

Les prothèses de hanche ou d'épaule sans ciment doivent être positionnées dans une orientation prédéterminée. En effet, le chirurgien n'a pas la possibilité de corriger l'orientation de la tige fémorale en per-opératoire lors de sa mise en place.

Dans la pratique courante pour les prothèses de hanche, le chirurgien résèque le col fémoral à une position déterminée. La section du col détermine le diamètre d'ouverture par laquelle l'implant ou la tige fémorale devront être introduites.

Dans les diverses et nombreuses prothèses de hanche existant, plusieurs solutions sont proposées, à savoir :
- lors de la mise en place de gros implants ou tiges fémorales, le chirurgien doit adapter la résection du col, ce qui oblige un retrait important de matière osseuse fragilisant la tenue de l'implant.
- lorsqu'on utilise des implants de forme dite anatomique, certaines zones osseuses sont enfoncées pour permettre l'introduction. Ces zones enfoncées ne sont plus en contact avec l'implant une fois la prothèse mise en place, ce qui a pour effet de diminuer le calage immédiat de la prothèse, induisant ainsi un risque de mobilité de l'implant.

C'est à ces inconvénients qu'entend plus particulièrement remédier la présente invention.

La tige suivant la présente invention a pour but de définir les zones privilégiées sur lesquelles elle sera en appui, en prenant en compte son introduction pour venir s'appuyer entièrement sur la surface osseuse.

A cet effet, la tige suivant l'invention présente dans sa partie métaphysaire un profil comme défini dans la revendication 1.

En outre, l'angle d'inclinaison de chacune des faces postérieure et antérieure par rapport à l'axe XX' de la diaphyse est compris de préférence entre 7° et 15°.

On remarque que la face postérieure de la partie métaphysaire présente une portion de cylindre ou un rayon de courbure de 2 000 mm pouvant varier de plus ou moins 500 mm.

On constate que la face antérieure de la partie métaphysaire présente une portion de cylindre ou un rayon de courbure de 240 mm pouvant varier de plus ou moins 40 mm.

La tige suivant la présente invention s'applique indépendament à des prothèses de hanche et à des prothèses d'épaule.

Le dessin annexé, donné à titre d'exemple, permettra de mieux comprendre l'invention, les caractéristiques qu'elle présente et les avantages qu'elle est susceptible de procurer :
Fig. 1 est une vue de face illustrant la tige suivant la présente invention et plus particulièrement l'aspect de surface de sa face postérieure.
Fig. 2 est une vue de profil de la tige suivant la présente invention.
Fig. 3 est une vue de face représentant la surface antérieure de la tige suivant l'invention.
Fig. 4 à 8 sont des coupes suivant IV-IV, V-V, VI-VI, VII-VII, VIII-VIII (fig. 1) illustrant le déplacement des centres de chaque rayon formant le profil de la face interne de la tige par rapport aux axes de référence de la diaphyse.

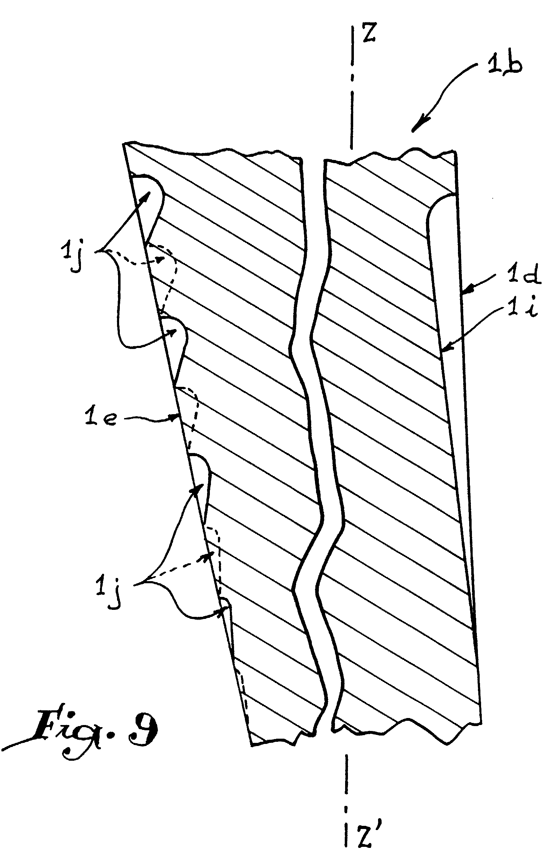

Fig. 9 est une coupe suivant IX-IX (fig. 1 et 3) montrant l'aspect de surface des faces postérieure et antérieure de la tige.

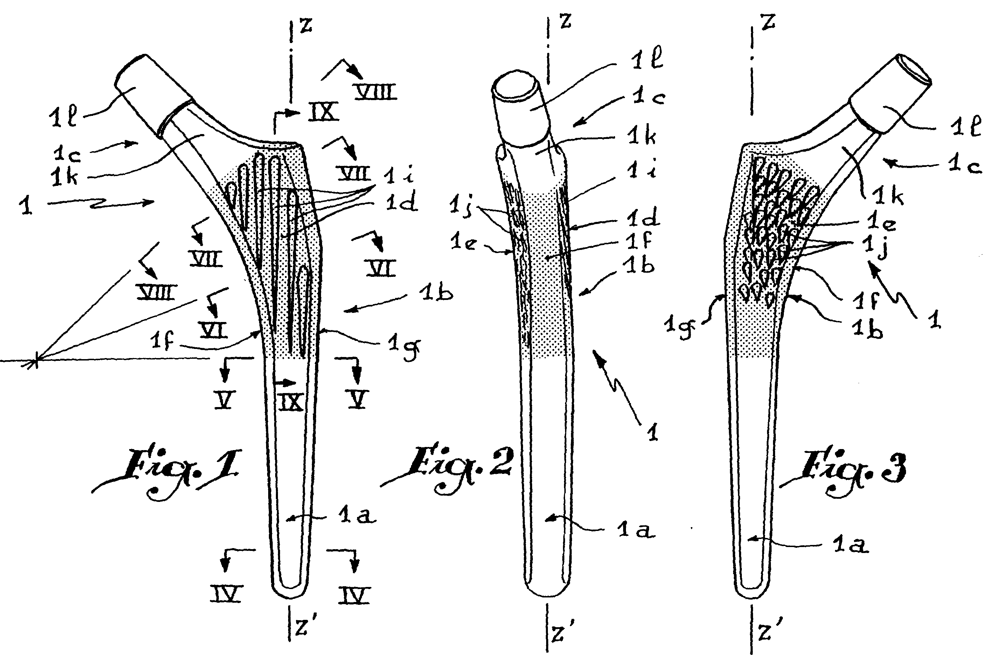

On a représenté en fig. 1 à 3 une tige 1 et notamment une tige fémorale d'une prothèse de hanche dont le profil extérieur est prévu pour permettre son introduction sans ciment à l'intérieur du canal médullaire du fémur (non représenté) d'un patient.

La tige fémorale 1 est représentée dans sa forme géométrique par rapport à des axes de référence XX' ; YY' et ZZ' qui sont ceux de la diaphyse d'un fémur. On note également que le plan frontal est définit par les axes XX'-ZZ', le plan sagital par les axes YY'-ZZ', tandis que l'axe diaphysaire est référencé ZZ' .

La tige fémorale 1 se compose de trois zones distinctes, c'est-à-dire d'une partie distale 1a formant l'extrémité inférieure de la tige, d'une partie métaphysaire ou médiane 1b et d'une partie extra-médullaire 1c constituant l'extrémité libre supérieure de la tige.

La tige fémorale 1 comporte une face postérieure 1d, une face antérieure 1e, une face interne 1f et une face externe 1g dont les profils de chacune varient suivant que l'on considère les parties distale 1a ou métaphysaire 1b.

La partie distale 1a présente suivant le plan frontal de fig. 1 et le plan sagittal de fig. 2 un profil conique d'inclinaison différente. En effet, les faces 1f et 1g ne sont pas inclinées du même angle que les faces 1d et 1e.

Dans la partie métaphysaire 1b, la tige fémorale 1 s'épaissit progressivement en gardant les faces postérieure 1d et antérieure 1e inclinées chacune par rapport à l'axe XX' de la diaphyse d'un angle différent qui est constant et compris entre 7 et 15° selon le profil anatomique désiré. Les faces postérieure 1d et antérieure 1e présentent par rapport à l'axe XX' de la diaphyse une inclinaison constante sur toute la hauteur de la tige 1 dont l'angle est compris entre 7 et 15° (fig. 4).

De plus, on constate que les faces 1d et 1e se prolongent dans la partie métaphysaire 1b par un profil en portion de cylindre suivant le plan sagittal indexé autour de l'axe ZZ' de l'angle précité de la face correspondante (fig. 2). La surface postérieure 1d peut être décrite, par exemple, par une portion de cylindre ou un rayon de courbure de 2 000 mm pouvant varier de plus ou moins 500 mm sans contrarier le profil anatomique de la tige fémorale 1. Egalement, la surface antérieure 1e peut être décrite, par exemple, par une portion de cylindre ou un rayon de courbure de 240 mm variant de plus ou moins 40 mm sans contrarier le profil de la tige fémorale 1.

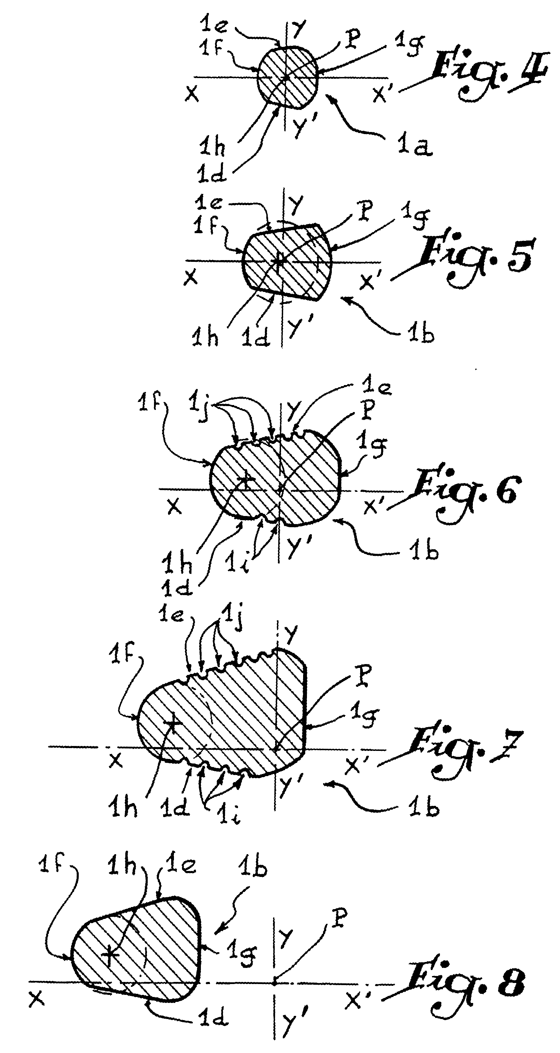

La face interne 1f se prolonge dans la partie métaphysaire 1b suivant un profil courbe comme représenté en fig. 1. La face interne 1f présente la même forme torique de rayon constant comme prévu dans la partie distale 1a. Par contre, l'ensemble des centres 1h des rayons formant le profil de la face interne 1f dans la partie métaphysaire 1b et à différents niveaux de celle-ci se déplacent latéralement et antérieurement par rapport au milieu P des axes XX' et YY' de la diaphyse comme représenté en fig. 5 à 8. On constate que ledit déplacement des centres 1h de chaque rayon formant le profil de la face interne 1f entraîne un décalage de l'ensemble de la tige fémorale 1 par rapport au centre P des axes XX', YY' de la diaphyse afin de lui donner un aspect particulier. La projection dans le plan frontal du profil de la face interne 1f est une portion de cercle. Cette projection est décrite par exemple par une portion de cercle de rayon 85 mm variant de plus ou moins 25 mm.

La face postérieure 1d comporte un aspect de surface en forme de rainures longitudinales 1i dont la profondeur augmente du bas vers le haut, de manière à lutter contre les efforts rotatoires auxquels serait soumise la tige fémorale 1. Chaque rainure 1i présente une forme allongée en goutte d'eau inversée, c'est-à-dire que la partie la plus large se trouve à proximité de la partie extra-médullaire 1c de la tige fémorale 1 (fig. 1).

La face antérieure 1e est recouverte également d'un aspect de surface en forme de goutte d'eau 1j dont les dimensions évoluent en profondeur, en largeur et en longueur de façon croissante du bas vers le haut, de manière à lutter contre les efforts d'enfoncement de la tige fémorale 1 et à créer des cavités de repousse osseuse variables en fonction des possibilités locales d'ostéogénèse. Les gouttes d'eau 1j sont de longueur très inférieure à celle des gouttes prévues sur la face postérieure 1d et sont également inversées de manière que la partie la plus large de la goutte 1j se trouve à proximité de la partie extra-médullaire 1c de la tige fémorale 1 (fig. 3).

La partie extra-médullaire 1c de la tige fémorale 1 se compose essentiellement d'un axe 1k se terminant par une partie conique 1l destinée à recevoir une tête fémorale prothétique non représentée. La géométrie de la partie extra-médullaire 1c de la tige fémorale 1 se définit en fonction de la longueur du col prothétique, du bras de levier défini par la distance du point extrême de la partie conique 1l à l'axe diaphysaire ZZ' et de l'angle d'antéversion défini par la projection dans le plan sagittal (fig. 2) de l'axe de la partie conique 1l par rapport à l'axe diaphysaire ZZ' .

On note que les paramètres varient entre les différentes tailles de tiges fémorales, de sorte que le bras de levier de la partie extra-médullaire 1c évolue selon une loi gaussienne tout en conservant un angle d'antéversion constant et une longueur de col variant par paliers en fonction de la partie intra-médullaire, elle-même définie par le diamètre d'alésage de la diaphyse du fémur.

Il est bien évident que la tige pour prothèse décrite ci-dessus peut s'appliquer par exemple aux prothèses d'épaule sans pour cela changer l'objet de la présente invention.

## Revendications

1. Tige pour prothèse, comprenant une partie diaphysaire ou distale (1a) ayant des axes de référence (X-X', Y-Y', ZZ') et des parties métaphysaire (1b) et extra-médullaire (1c), et comportant des faces postérieures (1d), antérieure (1e), interne (1f) et externe (1g), caractérisée en ce que dans la partie métaphysaire (1b) :
- la face interne (1f) a un profil arrondi qui varie par un déplacement latéral et antérieur des centres (1h) de chaque rayon, par rapport au milieu (P) des axes (X-X', Y-Y') de la diaphyse ; et
- les faces postérieure (1d) et antérieure (1e) sont inclinées par rapport à l'axe (X-X') de la diaphyse d'un angle différent, qui est constant sur toute la hauteur de la partie métaphysaire (1b) de la tige, et ont un rayon de courbure différent, de centre différent, défini dans le plan sagittal de ladite tige (1) qui est indexé autour de l'axe diaphysaire (ZZ') de l'angle précité de la face correspondante.

2. Tige pour prothèse suivant la revendication 1, caractérisée en ce que l'angle d'inclinaison de chacune des faces postérieure (1d) et antérieure (1e) par rapport à l'axe XX' de la diaphyse est compris entre 7 et 15°.

3. Tige pour prothèse suivant la revendication 1, caractérisée en ce que le prolongement de la face postérieure (1d) de la partie métaphysaire (1b) présente une portion de cylindre ou un rayon de courbure de 2 000 mm pouvant varier de plus ou moins 500 mm.

4. Tige pour prohèse suivant la revendication 1, caractérisée en ce que la face antérieure (1e) se prolongeant dans la partie métaphysaire (1b) présente une portion de cylindre ou un rayon de courbure de 240 mm pouvant varier de plus ou moins 40 mm.

5. Tige pour prothèse suivant la revendication 1, caractérisée en ce qu'elle comprend une partie distale (1a) de forme conique dont les faces postérieure (1d) et antérieure (1e) sont inclinées d'un angle différent de celui prévu pour les faces interne (1f) et externe (1g), une partie métaphysaire (1b) dont les faces postérieure (1d) et antérieure (1e) se prolongent suivant un rayon de courbure différent, de centre différent, tandis que la face interne (1f) se poursuit suivant un profil courbe qui est déterminé en fonction du décalage angulaire des centres (1h) de chaque rayon formant son profil arrondi qui en projection dans le plan frontal représente un arc de cercle et une partie extra-médullaire (1c) comportant essentiellement un axe (1k) se terminant par une partie conique (1l) destinée à recevoir une tête prothétique.

6. Tige pour prothèse suivant la revendication 1, caractérisée en ce que la face postérieure (1d) comprend dans la partie métaphysaire (1b) une série de rainures (1i) en forme de gouttes d'eau inversées dont la profondeur augmente du bas vers le haut de la tige (1) de manière à lutter contre les efforts rotatoires auxquels elle serait soumise.

7. Tige pour prothèse suivant la revendication 6, caractérisée en ce que la partie la plus large des rainures (1i) est dirigée du côté de la partie extra-médullaire de la tige (1).

8. Tige pour prothèse suivant la revendication 1, caractérisée en ce que la face antérieure (1e) est pourvue d'un certain nombre de gouttes d'eau inversées (1j) dont les dimensions évoluent en profondeur, en largeur, et en longueur de façon croissante du bas vers le haut de la tige fémorale (1) de manière à lutter contre les efforts d'enfoncement de ladite tige et à créer des cavités de repousse osseuse variables en fonction des possibilités locales d'ostéogénèse.

9. Tige pour prothèse suivant la revendication 8, caractérisée en ce que les gouttes d'eau inversées (1j) sont disposées de manière que leur partie la plus large soit tournée du côté de la partie extramédullaire (1c) de la tige (1).

10. Tige fémorale de prothèse de hanche, caractérisée en ce qu'elle comprend un profil déterminé suivant l'une quelconque des revendications 1 à 9.

11. Tige humérale de prothèse d'épaule, caractérisée en ce qu'elle comprend un profil déterminé suivant l'une quelconque des revendications 1 à 9.

## Patentansprüche

1. Prothesenschaft, der einen diaphysären oder distalen Abschnitt (1a) mit Bezugsachsen (X-X', Y-Y', Z-Z') und einen metaphysären (1b) und einen äußeren medulären (1c) Abschnitt aufweist sowie eine vordere Fläche (1d), eine hintere Fläche (1e), einen Innenfläche (1f) und eine Außenfläche (1g) aufweist,
dadurch **gekennzeichnet**,
daß , daß in dem metaphysären Abschnitt (1b)
- die Innenfläche (1f) ein rundes Profil besitzt, daß variiert durch einen Versatz zur Seite und nach vorne der Mittelpunkte eines jeden Radius zur Mitte (P) der Achsen (X-X', Y-Y') der Diaphyse und
- die hintere Fläche (1 d) und die vordere Fläche (1e) zur Achse (X-X') der Diaphyse jeweils einen unterschiedlichen Neigunswinkel aufweisen, der über die gesamte Höhe des metaphysären Abschnitts (1b) des Schaftes (1) konstant bleibt und einen abweichenden Krümmungsradius mit einem abweichenden Mittelpunkt besitzen, der in der Sagittalebene des Schaftes (1) liegt, die um die diaphysäre Achse (Z-Z') um den genannten Winkel zu der entsprechenden Fläche ausgerichtet ist.

2. Prothesenschaft nach Anspruch 1,
dadurch **gekennzeichnet**,
daß der Neigungswinkel sowohl der hinteren Fläche (1d) als auch der vorderen Fläche (1e) zur Achse (XX') der Diaphyse zwischen 7° und 15° beträgt.

3. Prothesenschaft nach Anspruch 1,
**dadurch gekennzeichnet**,
daß die Verlängerung der hinteren Fläche (1d) des metaphysären Abschnitts (1b) einen zylindrischen Abschnitt oder einen Krümmungsradius von 2000 mm aufweist der mehr oder weniger und 500 mm variieren kann.

4. Prothesenschaft nach Anspruch 1,
dadurch **gekennzeichnet**,
daß sich die vordere Fläche (1e) in den metaphysären Abschnitt (1b) verlängert und einen zylindrischen Abschnitt oder einen Krümmungsradius von 240 mm aufweist, der mehr oder weniger um 40 mm variieren kann.

5. Prothesenschaft nach Anspruch 1,
dadurch **gekennzeichnet**,
daß dieser einen kegelförmigen distalen Abschnitt (1a), dessen hintere Fläche (1d) und vordere Fläche (1e) um einen Winkel geneigt sind, der von demjenigen Winkel abweicht, der für die Innenflächen (1f) und Außenflächen (1g) vorgesehen ist, und einen metaphysären Abschnitt (1b) aufweist, dessen hintere Fläche (1d) und vordere Fläche (1e) sich ausgehend von einem abweichenden Mittelpunkt entlang einem abweichenden Krümmungsradius verlängern, wohingegen sich die Innenfläche (1f) in einem gekrümmten Profil fortsetzt, das sich in Abhängigkeit des Winkelversatzes der Mittelpunkte (1h) eines jeden Radius unter Ausbildung eines runden Profils bestimmt, das in der frontalen Projektionsebene einen Kreisbogen und einen äußeren medullären Abschnitt (1c) darstellt, die im wesentlichen eine Achse (1k) aufweisen, die in einem kegelförmigen Abschnitt (1l) zur Aufnahme des Prothesenkopfes endet.

6. Prothesenschaft nach Anspruch 1,
dadurch **gekennzeichnet**,
daß die hintere Fläche (1d) in ihrem metaphysären Abschnitt (1b) mehrere Rillen (1i) in der Form umgedrehter Wassertropfen aufweist, deren Tiefe im Schaft (1) von unten nach oben ansteigt, um den Rotationskräften, denen dieser ausgesetzt ist, entgegenzuwirken.

7. Prothesenschaft nach Anspruch 6,
dadurch **gekennzeichnet**,
daß der größere Bereich der Rillen (1i) zur Seite des äußeren medullären Abschnitts (1c) des Schafts (1) gerichtet ist.

8. Prothesenschaft nach Anspruch 1,
dadurch **gekennzeichnet**,
daß die vordere Fläche (1e) mit einer bestimmten Anzahl an umgedrehten wassertropfförmigen Rillen (1j) versehen ist, deren Dimensionen sich in Tiefe, Größe und Länge von unten nach oben im Femurschaft (1) halbmondförmig fortsetzen, um den Senkkräften des Schaftes (1) entgegenzuwirken und um unterschiedliche Kavitäten für das Nachwachsen des Knochengewebes in Abhängigkeit der lokalen Gegebenheiten der Knochenbildung auszubilden.

9. Prothesenschaft nach Anspruch 8,
dadurch **gekennzeichnet**,
daß die umgedrehten wassertropfförmigen Rillen (1j) so ausgerichtet sind, daß deren größerer Abschnitt zur Seite des äußeren medullären Abschnitts (1c) des Schafts (1) gedreht ist.

10. Femurschaft einer Hüftprothese
**gekennzeichnet** durch
ein Profil, das sich nach einem der Ansprüche 1 bis 9 bestimmt.

11. Humerusschaft einer Schulterprothese
**gekennzeichnet** durch
ein Profil, das sich nach einem der Ansprüche 1 bis 9 bestimmt.

## Claims

1. Shaft for a prosthesis comprising a diaphysial or distal part (1a) with reference axes (X-X', Y-Y', Z-Z') and methaphysial (1b) and extra-medullar (1c) parts, and provided with posterior (1d), anterior (1d), internal (1f) and external (1g) faces, characterized in that, in the methaphysial part (1b), :
- the internal face (1f) has a rounded profile which varies by lateral and anterior displacement of the centers of each radius, with respect to the middle (P) of the axes (X-X', Y-Y') of the diaphysis ; and
- the posterior (1d) and anterior (le) faces are inclined with respect to the axis (X-X') of the diaphysis of a different angle, which is constant over the entire height of the metaphysial part (1b) of the shaft, and have a different radius of curvature, with a different center, defined in the sagittal plane of said shaft (1) which is indexed about the diaphysial axis (Z-Z') of the above mentioned angle of the corresponding face.

2. Shaft for a prosthesis according to claim 1, characterised in that the angle of inclination of each of the posterior (1d) and anterior (1e) faces with respect to the axis XX' of the diaphysis is between 7 and 15°.

3. Shaft for a prosthesis according to claim 1, characterised in that the continuation of the posterior face (1d) of the metaphysial part (1b) presents a portion of a cylinder or a radius of curvature of 2000 mm which may vary by more or less than 500 mm.

4. Shaft for a prosthesis according to claim 1, characterised in that the anterior face (1e) which continues into the metaphysial part (1b) presents a portion of a cylinder or a radius of curvature of 240 mm which may vary by more or less than 40 mm.

5. Shaft for a prosthesis according to claim 1, characterised in that it comprises a cone-shaped distal part (1a) whose posterior (1d) and anterior (1e) faces are inclined by a different angle from that provided on the inner (1f) and outer (1g) faces, a metaphysial part (1b) whose posterior (1d) and anterior (1e) faces continue along a different radius of curvature, with a different centre, while the inner (1f) face follows a curved profile which is set in response to the angular offset of the centres (1b) of each radius forming the rounded profile thereof which in projection in the frontal plane represents an arc of a circle and an extra-medullary part (1c) which essentially includes an axis (1k) which terminates in a conical part (1l) which is designed to receive a prosthetic head.

6. Shaft for a prosthesis according to claim 1, characterised in that in the metaphysial part (1b) the posterior face (1d) incorporates a series of grooves (1i) in the shape of inverted water droplets of a depth that increases from the bottom to the top of the shaft (1) in such a way as to resist the rotary stresses to which it would be subjected.

7. Shaft for a prosthesis according to claim 6, characterised in that the broadest part of the grooves (1i) is on the side nearest the extra-medullary part of the shaft (1).

8. Shaft for a prosthesis according to claim 1, characterised in that the anterior face (1e) is provided with a number of inverted water droplets (1j) of dimensions that develop depthwise, widthwise and lengthwise in a manner criss-crossing from the bottom to the top of the femoral shaft (1) so as to resist the forces seeking to push down the shaft and to create variable bone regrowth cavities in response to local possibilities for bone formation.

9. Shaft for a prosthesis according to claim 8, characterised in that the inverted water droplets (1j) are arranged in such a way that the widest part thereof is situated nearest the extra-medullary part (1c) of the shaft (1).

10. Femoral shaft for a hip prosthesis, characterised in that it has a given profile according to any one of claims 1 to 9.

11. Humeral shaft for a shoulder prosthesis, characterised in that it has a given profile according to any one of claims 1 to 9.
